# EUROPEAN PATENT SPECIFICATION

(11) **EP 1 803 440 B1**
(45) Date of publication and mention of the grant of the patent: **02.06.2010**
(21) Application number: 05802903.4
(22) Date of filing: 13.10.2005
(51) Int. Cl.: A61K 9/50, A61K 31/20, A61P 3/06, A61K 9/16, A61K 9/48

(54) **PHARMACEUTICAL FORMULATION COMPRISING MICROCAPSULES OF STATINS SUSPENDED IN ALKYL ESTERS OF POLYUNSATURATED FATTY ASIDS (PUFA)**
PHARMAZEUTISCHE FORMULIERUNG MIT STATINMIKROKAPSELN IN ALKYLESTERN MEHRFACH UNGESÄTTIGTER FETTSÄUREN
FORMULATION PHARMACEUTIQUE COMPRENANT DES MICROCAPSULES DE STATINES EN SUSPENSION DANS DES ESTERS ALKYLIQUES D'ACIDES GRAS POLYINSATURES (PUFA)

(30) Priority: 19.10.2004 ES 200402492
(43) Date of publication of application: 04.07.2007
(73) Proprietor: GP Pharm S.A., 08777 Sant Quinti Mediona Barcelona (ES); Defiante Farmacêutica S.A., 9000 082 Funchal Madeira (PT)
(72) Inventor: CARMINATI, Paolo, I-20121 Milán (IT); PARENTE, Antonio, E-08960 San Just Desvern Barcelona (ES)
(74) Representative: Carvajal y Urquijo, Isabel
(86) International application number: PCT/ES2005/000542
(87) International publication number: WO 2006/045865

(56) References cited:
- WO-A-03/103640
- WO-A1-02/100394
- WO-A2-02/43659
- US-A- 5 861 399
- SALVI A ET AL: "Effects of fish oil on serum lipids and lipoprotein(A) levels in heterozygous familial hypercholesterolemia" CURRENT THERAPEUTIC RESEARCH, EXCERPTA MEDICA, TRENTON, NJ, US, vol. 53, no. 6, 1 January 1993 (1993-01-01), pages 717-721, XP002424143 ISSN: 0011-393X

## Description

### State of the Art

The present invention describes a new pharmaceutical composition for the administration of statins and the process of preparing them.

The efficacy of the statins in the primary and secondary prevention of cardiovascular diseases has been demonstrated in a number of clinical studies. Recent evidence suggests that the clinical benefit obtained with therapy with statins could be related to a reduction of systemic inflammatory markers (Ridker P.M., et al.; N. Engl, J. Med. 344:1959-65, 2001) more than to the reduction of cholesterol level. Even though it has not been possible to prove that there is a direct relation of the anti-inflammatory mechanism of statins in the reduction of cardiovascular events, recent studies have shown that the treatment with statins improves plaque stability and reduces the arterial inflammatory reaction in patients subjected to endarterectomy (Crisby M., et. al.; Circulation 103:926-33, 2001). In addition, therapy with statins in experimental models determines the reduction of expressors of the inflammatory lesion, such as for example of the macrophage infiltration content (Van der Wal A.C., et a).; Circulation 89:36-44, 1994), of the release of VCAM-1, of interleukin-1β and of tissue factor in the arteriosclerotic lesion (Sukhova GK, et al.; Arterioscler Thromb Vaso Biol 22:1452-8, 2002).

Omega-3 type polyunsaturated fatty acids (Ω-3 PUFA) have demonstrated a beneficial effect in the prevention of cardiovascular events (Bucher HC, et al.; Am. J. Med. 2002; 112:298-304), possibly by means of an antiinflammatory, antithrombotic and antiarrhythmic mechanism (Sethi S, et al.; Blood 2002:100:-1340-6; Billman GE, et al.; Circulation 1999: 99:2452-7).

It is also disclosed in the state of the art (Salvi A., et al.; Curr. Ther. Res. 53(6): 717-21) a combination therapy of simvastatin with fish oil, where both drugs are administered in two separated oral capsules. There is no indication in this document about a common capsule for both drugs together.

Different patents have been published which describe pharmaceutical formulations of statins, such as for example:
US 5,180,589 or US 5,356,896 which describe pharmaceutical composition forms for the stabilization of statins at low pH.
US 6,235,311 describes a pharmaceutical composition combining a statin and aspirin.
US 5,225,202 describes a pharmaceutical composition of statins in the form of pellets with an enteric coating so as to protect the product at low pH.
WO00/76482, WO00/57918 and WO00/57859 describe pharmaceutical compositions formed by lipid regulating agents in oils or in surfactants.
WO02/100394 and WO03/103640 describe pharmaceutical compositions formed by pure statin nanoparticles without any protective coating dispersed in pharmaceutically acceptable oils, however this type of formulations have stability problems for statins if the temperature of preparing the system exceeds 40°C, as is the case for most oral preparations. Particularly in WO03/103640, the pharmaceutically acceptable oil can be a fish oil. However, the stability problems of the pharmaceutical composition disclosed in WO02/100394 are not mentioned, neither solved, in WO03/10364.

### Summary of the Invention

As a result of the research carried out by the inventors, a new formulation has been developed consisting of a microcapsule suspension of statins in alkyl esters of PUFA in which the statins are isolated from contact with the alkyl ester of PUFA by means of a polymeric membrane that can be easily disintegrated in the gastrointestinal medium.

This coating provides stabilization of the statin, eliminating the occurrence of degradation products of the statin during the processes of preparing the microcapsule suspension and of incorporating the mentioned microcapsule suspension of statins in alkyl esters of PUFA in final system of administering the product (soft gelatin capsules, hard gelatin capsules, tablets, granules, etc.), even though these processes are carried out at a temperature exceeding 40°C.

### Detailed Description of the Invention

A new preparation for statins has been developed which surprisingly allows avoiding the problems of degradation that statins have when they are formulated in the presence of oils with a high content of alkyl esters of PUFA.

Therefore, according to a first essential aspect, the present invention relates to a pharmaceutical formulation characterized in that it is made up of a suspension comprising an oil exceeding 60% in alkyl esters of polyunsaturated fatty acid (PUFA) and microcapsules comprising at least one polymer and a statin.

Said alkyl esters of polyunsaturated fatty acid (PUFA) preferably belong to the Omega3 series, they are more preferably selected from the group consisting of the eicosapentaenoic acid, docosahexaenoic acid or mixtures thereof.

According to a preferred embodiment according to the present invention, the alkyl ester of PUFA is selected from the group consisting of ethyl, methyl, propyl, butyl esters, or mixtures thereof.

Preferably, the statins selected from the group consisting of simvastatin, lovastatin, fluvastatin, atorvastatin, cerivastatin, pravastatin, rosuvastatin or mixtures thereof.

The polymer coating the microcapsules of statins is preferably selected from the group consisting of polyesters, polyacrylates, polycyanoacrylates, polysaccharides, polyethylene glycol, or mixtures thereof. More preferably, the polymer coating the microcapsules of statins is selected from the group consisting of gelatin, carboxymethylcellulose, alginates, carrageenans, pectins, ethyl cellulose hydroxypropyl methylcellulose, cellulose acetophthalate, hydroxypropyl methylcellulose phthalate, methylacrylic acid copolymers (Eudragit® L and S), dimethylaminoethylmethacrylate copolymers (Eudragit® E), the trimethylammoniumethylmethacrylate copolymers (Eudragit® RL and RS), polymers and copolymers of lactic and glycolic acids or mixtures thereof.

Optionally, a pharmaceutical formulation according to the present invention comprises an antioxidant, preferably vitamin E acetate. According to a preferred embodiment according to the present invention, the pharmaceutical formulation comprises carnitine.

Preferably, the microcapsules represent between 1% and 60% of the total weight of the pharmaceutical formulation according to the present invention, and the amount of statin incorporated in said microcapsules is comprised between 1% and 80% by weight, preferably between 1 and 40% by weight in relation to the total weight of the microcapsules.

According to a preferred embodiment according to the present invention, the polymer comprises a plasticizer additive, preferably those plasticizers selected from the group consisting of triethyl citrate, butyl phthalate or mixtures thereof. Other technical additives of the polymer can optionally be incorporated which improve or facilitate the encapsulation process, such as, for example, fluidizing agents, preferably talc.

The ratio between eicosapentaenoic acid and docosahexaenoic acid is preferably comprised between 0.5 and 2.

According to a preferred embodiment of the present invention, the microcapsule suspension is encapsulated by soft gelatin capsules for oral administration. Said soft gelatin capsules preferably have an enteric coating.

The preparation of the microcapsules can be carried out following any of the methods described in the literature. By way of description and without being limited thereto, the different processes of obtaining microcapsules could be grouped into the following categories:
A) Simple coacervation methods:
   A solution of the polymer together with the possible additives of the polymer in a suitable solvent is prepared. The drug to be encapsulated is suspended in said solution of the polymer and a non-solvent of the polymer is added so as to force the deposit of the polymer on the drug crystals. Examples of these processes can be found in patent documents such as ES 2009346, EP 0 052 510, or EP 0 346 879.
B) Complex coacervation method
   This method is based on the interaction between two colloids having opposite electric charges so as to generate an insoluble complex that is deposited on the particles of the drug to be encapsulated, forming a membrane that will isolate the drug. Examples of these processes can be found in patent documents such as GB 1393805.
C) Double emulsion methods:
   The drug to be encapsulated is dissolved in water or in a solution of some other coadyuvant and is emulsified in a solution of the polymer and additives in a suitable solvent, such as for example dichloromethane. The resulting emulsion is in turn emulsified in water or in an aqueous solution of an emulsifying agent, such as polyvinyl alcohol. Once this second emulsion is carried out the solvent in which the polymer and the plasticizer were dissolved in is eliminated by means of evaporation or extraction. The resulting microcapsules are obtained directly by filtration or evaporation. Examples of these processes can also be found in patent documents such as US 4,652,441.
D) Simple emulsion methods:
   The drug to be encapsulated, the polymer and the additives are dissolved together in a suitable solvent. This solution is emulsified in water or in an emulsifier solution, such as polyvinyl alcohol, and the organic solvent is eliminated by evaporation or by extraction. The resulting microcapsules are recovered by filtration or drying. Examples of these processes can also be found in patent documents such as US 5,445,832
E) Solvent evaporation methods:
   The drug to be encapsulated, the polymer and additives are dissolved together in a suitable solvent. This solution is evaporated and the resulting residue is micronized to the suitable size. Examples of this process can also be found in patent documents such as GB 2,209,937.

### EXAMPLES:

### Example no. 1: Preparation of microcapsules of simvastatin with gelatin and carboxymethyl cellulose by means of complex coacervation processes.

Solution A: A 1% solution of gelatin in water is prepared and the pH is adjusted so that it is equal to or greater than 7.

Solution B: Another 1% solution of sodium carboxymethyl cellulose in water is prepared and the pH is adjusted so that it is equal to or greater than 7.

100 mL of solution A and 100 mL of solution B are mixed and heated to 40°C. 1.2 g of powder simvastatin are dispersed in the mixture. When all the powder is dispersed and no lumps are observed, the pH is adjusted to 4.0 by means of adding acetic acid. It is maintained under stirring for 1 hour at 40°C and then the solution is cooled to 10°C, this temperature being maintained for another hour. 1 mL of a 50% solution of glutaraldehyde in water is added.

The resulting suspension is dried by means of spray drying, obtaining a microcapsules powder containing 37% of simvastatin.

This microcapsule powder is directly dispersed in oil containing 88% ethyl ester of PUFA with an eicosapentaenoic acid (EPA)/docosahexaenoic acid (DHA) ratio of 1.2.

### Example no. 2: Preparation of microcapsules of simvastatin with gelatin by means of simple coacervation processes.

A 1 % solution of gelatin in water is prepared.

100 mL of this solution are taken and 1 g of powder simvastatin is dispersed therein. Once all the simvastatin has been dispersed 30 mL of a saturated solution of sodium sulfate in water are added. It is maintained under stirring for 1 hour and 0.5 mL of a 50% solution of glutaraldehyde in water are added.

The formed microcapsules are collected by filtration, washed with water and dried in a vacuum oven. The simvastatin content of these microcapsules is 45%.

The resulting microcapsule powder is dispersed directly in oil containing 70% of methyl ester of PUFA with an EPA/DHA ratio of 0.8.

### Example no. 3: Preparation of microcapsules of lovastatin with polyethylene glycol.

A 10% solution of polyethylene glycol in water with molecular weight 35000 (PEG-35000) is prepared.

6 g of lovastatin are dispersed in this solution by means of intense stirring.

When a fine dispersion without lumps has been obtained, the solution is dried by means of spray drying.

The obtained microcapsule powder has a 40% lovastatin concentration and is dispersed directly in oil containing 85% of ethyl ester of PUFA with an EPA/DHA ratio of 1.

### Example no. 4: Preparation of microcapsules of simvastatin with cellulose acetophthalate.

A 2% solution of sodium acetophthalate in water is prepared. 5 g of simvastatin powder are suspended in 100 mL of this solution. The resulting suspension is dried by means of spray drying.

The obtained microcapsule powder is dispersed directly in oil containing 85% of ethyl ester of PUFA with an EPA/DHA ratio of 0.5.

### Example no. 5: Preparation of microcapsules of simvastatin with poly(lactic-glycolic acid) (PLGA) copolymer and vitamin E. Simple emulsion method (oil in water)

Solution A: A 10% solution of PLGA in dichloromethane (DCM) with intrinsic viscosity (I.V.) of 0.17 and lactic/glycolic ratio of 1/1 is prepared.

Solution B: 5 g of simvastatin and 1 g of vitamin E acetate are dissolved in 100 mL of solution A.

Solution C: A 1 % solution of polyvinyl alcohol (PVA) in water is prepared.

100 mL of solution B are added slowly and under intense stirring to 1000 mL of solution C until obtaining a milky emulsion.

While stirring is maintained, a nitrogen current is passed through the previous emulsion for two hours to eliminate most of the DCM.

Then the resulting suspension is frozen and lyophilized. A powder is obtained which is washed with abundant water to eliminate the excess PVA and is dried under reduced pressure.

The resulting powder is dispersed directly in oil containing 85% of ethyl ester of PUFA with an EPA/DHA ratio of 1.5.

### Example no. 6: Preparation of microcapsules of simvastatin with carnitine and polyethylene glycol

A 10% solution of polyethylene glycol in water with molecular weight 35000 (PEG-35000) is prepared.

5 g of simvastatin and 1 g of carnitine are dispersed in this solution by means of intense stirring. When a fine dispersion without lumps has been obtained the solution is dried by means of spray drying.

The obtained microcapsule powder is dispersed directly in oil containing 85% of ethyl ester of PUFA with an EPA/DHA ratio of 1.

### Example no. 7: Preparation of microcapsules of simvastatin with carnitine, vitamin E and PLGA, triple emulsion method (water in oil and in water.)

Solution A: A 10% solution of carnitine in water is prepared.

Solution B: A 10% solution of PLGA in dichloromethane (DCM) with intrinsic viscosity (I.V.) of 0.17 and lactic/glycolic ratio 1/1 is prepared.

Solution C. 10 g of simvastatin and 1 g of vitamin E acetate are dissolved in 100 mL of solution B.

20 mL of solution A are emulsified in solution C by means of intense stirring with an Ultra Turrax homogenizer. The resulting emulsion is in turn emulsified in 1000 mL of a 1 % solution of PVA in water.

While stirring is maintained, a nitrogen current is passed through the previous emulsion for two hours to eliminate most of the DCM.

Then the resulting suspension is frozen and lyophilized. A microcapsule powder is obtained which is washed with abundant water to eliminate the excess PVA and is dried under reduced pressure in a vacuum oven.

The resulting microcapsule powder has a simvastatin concentration of 25% and is dispersed directly in oil containing 85% of ethyl ester of PUFA with an EPA/DHA ratio of 1.2.

### Example no. 8: Preparation of microcapsules of simvastatin and a methacrylic acid copolymer

10 g of simvastatin are suspended in 100 mL of a suspension of Eudragit FS 30D^{®} (30% suspension in water of copolymers of methacrylic acid, methyl methacrylic acid and methyl acrylate) until obtaining a fine suspension. Triethyl citrate (plasticizer of the polymer) is added to this suspension up to a concentration of 5%.

The resulting suspension is dried by means of spray drying.

The resulting microcapsule powder is dispersed directly in oil containing 85% of ethyl ester of PUFA with an EPA/DHA ratio of 1.2.

## Claims

1. A pharmaceutical formulation **characterized in that** it is made up of a suspension comprising an oil, exceeding 60% in alkyl esters of polyunsaturated fatty acid (PUFA) and microcapsules comprising at least one polymer and a statin, said statin being isolated from the contact with said alkyl esters of PUFA by a polymeric membrane that can be easily disintegrated in the gastrointestinal medium.

2. A pharmaceutical formulation according to claim 1, **characterized in that** the PUFA belongs to the Omega3 series.

3. A pharmaceutical formulation according to any of the previous claims,
**characterized in that** the PUFA is selected from the group consisting of eicosapentaenoic acid, the docosahexaenoic acid or mixtures thereof.

4. A pharmaceutical formulation according to any of the previous claims,
**characterized in that** the alkyl ester of PUFA is selected from the group consisting of ethyl, methyl, propyl, butyl esters or mixtures thereof.

5. A pharmaceutical formulation according to any of the previous claims,
**characterized in that** the statins are selected from the group consisting of simvastatin, lovastatin, fluvastatin, atorvastatin, cerivastatin, pravastatin, rosuvastatin or mixtures thereof.

6. A pharmaceutical formulation according to any of the previous claims,
**characterized in that** the polymer coating the microcapsules of statins is selected from the group consisting of polyesters, polyacrylates, polycyanoacrylates, polysaccharides, polyethylene glycol, or mixtures thereof.

7. A pharmaceutical formulation according to any of the previous claims,
**characterized in that** the polymer coating the microcapsules of statins is selected from the group consisting of gelatin, carboxymethylcellulose, alginates, carrageenans, pectins, ethyl cellulose, hydroxypropyl methylcellulose, cellulose acetophthalate, hydroxypropyl methylcellulose phthalate, methacrylic acid copolymers, dimethylaminoethylmethacrylate copolymers, trimethylammoniumethylmethacrylate copolymers, lactic and glycolic acid polymers and copolymers or mixtures thereof.

8. A pharmaceutical formulation according to any of the previous claims,
**characterized in that** it further comprises an antioxidant

9. A pharmaceutical formulation according to claim 8, **characterized in that** said antioxidant is vitamin E acetate.

10. A pharmaceutical formulation according to any of the previous claims,
**characterized in that** it further comprises carnitine.

11. A pharmaceutical formulation according to any of the previous claims,
**characterized in that** the microcapsules represent between 1% and 60% of the total weight of the formulation.

12. A pharmaceutical formulation according to any of the previous claims,
**characterized in that** the amount of statin incorporated in the microcapsules is comprised between 1% and 80% by weight in relation to the total weight of the microcapsules.

13. A pharmaceutical formulation according to claim 12, **characterized in that** said amount of statin incorporated in the microcapsules is comprised between 1 and 40% by weight in relation to the total weight of the microcapsules.

14. A pharmaceutical formulation according to any of the previous claims
**characterized in that** it further comprises a plasticizer additive of the polymer

15. A pharmaceutical formulation according to claim 14, **characterized in that** said plasticizer additive is selected from the group consisting on triethyl citrate, butyl phthalate and mixtures thereof.

16. A pharmaceutical formulation according to any of claims 3-15,
**characterized in that** the ratio between eicosapentaenoic acid and docosahexaenoic acid is comprised between 0.5 and 2.

17. Any formulation of the previous claims, **characterized in that** the microcapsule suspension is encapsulated by soft gelatin capsules for oral administration.

18. A formulation according to claim 17, **characterized in that** the soft gelatin capsules have an enteric coating.

## Patentansprüche

1. Pharmazeutische Formulierung, **dadurch gekennzeichnet, dass** sie aus einer Suspension aufgebaut ist, welche ein Öl, das mehr als 60 % Alkylester mehrfach ungesättigter Fettsäuren (Polyunsaturated Fatty Acids, PUFA) aufweist, und Mikrokapseln umfasst, die mindestens ein Polymer und ein Statin umfassen, wobei das Statin durch eine Polymermembran, die im gastrointestinalen Medium leicht zersetzt werden kann, gegen einen Kontakt mit den PUFA-Alkylestern isoliert ist.

2. Pharmazeutische Formulierung nach Anspruch 1, **dadurch gekennzeichnet, dass** die PUFA zur Omega-3-Serie gehört.

3. Pharmazeutische Formulierung nach einem der vorhergehenden Ansprüche, **dadurch gekennzeichnet, dass** die PUFA aus der Gruppe ausgewählt ist, die aus Eicosapentaensäure, Docosahexaensäure und Gemischen davon besteht.

4. Pharmazeutische Formulierung nach einem der vorhergehenden Ansprüche, **dadurch gekennzeichnet, dass** der Alkylester der PUFA aus der Gruppe ausgewählt ist, die aus Ethyl-, Methyl-, Propyl-, Butylestern und Gemischen davon besteht.

5. Pharmazeutische Formulierung nach einem der vorhergehenden Ansprüche, **dadurch gekennzeichnet, dass** die Statine aus der Gruppe ausgewählt sind, die aus Simvastatin, Iovastatin, Fluvastatin, Atorvastatin, Cerivastatin, Pravastatin, Rosuvastatin und Gemischen davon besteht.

6. Pharmazeutische Formulierung nach einem der vorhergehenden Ansprüche, **dadurch gekennzeichnet, dass** das Polymer, mit welchem die Mikrokapseln der Statine beschichtet sind, aus der Gruppe ausgewählt ist, die aus Polyestern, Polyacrylaten, Polycyanacrylaten, Polysacchariden, Polyethylenglykol und Gemischen davon besteht.

7. Pharmazeutische Formulierung nach einem der vorhergehenden Ansprüche, **dadurch gekennzeichnet, dass** das Polymer, mit welchem die Mikrokapseln der Statine beschichtet sind, aus der Gruppe ausgewählt ist, die aus Gelatine, Carboxymethylcellulose, Alginaten, Carrageenanen, Pektinen, Ethylcellulose, Hydroxypropylmethylcellulose, Celluloseacetophthalat, Hydroxypropylmethylcellulosephthalat, Methacrylsäure-Copolymeren, Dimethylaminoethylmethacrylat-Copolymeren, Trimethylammoniummethylmethacrylat-Copolymeren, Milchsäure- und Glykolsäure-Polymeren und Copolymeren oder Gemischen davon besteht.

8. Pharmazeutische Formulierung nach einem der vorhergehenden Ansprüche, **dadurch gekennzeichnet, dass** sie ferner ein Antioxidationsmittel umfasst.

9. Pharmazeutische Formulierung nach einem der vorhergehenden Ansprüche, **dadurch gekennzeichnet, dass** es sich bei dem Antioxidationsmittel um Vitamin-E-acetat handelt.

10. Pharmazeutische Formulierung nach einem der vorhergehenden Ansprüche, **dadurch gekennzeichnet, dass** sie ferner Carnitin umfasst.

11. Pharmazeutische Formulierung nach einem der vorhergehenden Ansprüche, **dadurch gekennzeichnet, dass** die Mikrokapseln 1% bis 60% der Gesamtmasse der Formulierung ausmachen.

12. Pharmazeutische Formulierung nach einem der vorhergehenden Ansprüche, **dadurch gekennzeichnet, dass** die Menge des Statins, welches in die Mikrokapseln eingebracht ist, 1% bis 80% der Gesamtmasse der Mikrokapseln ausmacht.

13. Pharmazeutische Formulierung nach Anspruch 12, **dadurch gekennzeichnet, dass** die Menge des Statins, welches in die Mikrokapseln eingebracht ist, 1% bis 40% der Gesamtmasse der Mikrokapseln ausmacht.

14. Pharmazeutische Formulierung nach einem der vorhergehenden Ansprüche, **dadurch gekennzeichnet, dass** sie ferner einen Weichmacher-Additiv für das Polymer umfasst.

15. Pharmazeutische Formulierung Anspruch 14, **dadurch gekennzeichnet, dass** das Weichmacher-Additiv aus der Gruppe ausgewählt ist, die aus Triethylcitrat, Butylphthalat und Gemischen davon besteht.

16. Pharmazeutische Formulierung nach einem der Ansprüche 3 bis 15, **dadurch gekennzeichnet, dass** das Verhältnis zwischen Eicosapentaensäure und Docosahexaensäure 0,5 bis 2 beträgt.

17. Formulierung nach einem der vorhergehenden Ansprüche, **dadurch gekennzeichnet, dass** die Mikrokapselsuspension in Weichgelatinekapseln zur oralen Verabreichung verkapselt ist.

18. Formulierung nach Anspruch 17, **dadurch gekennzeichnet, dass** die Weichgelatinekapseln eine magensaftresistente Beschichtung aufweise.

## Revendications

1. Formulation pharmaceutique **caractérisée en ce qu'**elle est constituée d'une suspension comprenant une huile, dépassant 60 % en esters d'alkyle d'acide gras polyinsaturé (PUFA), et des microcapsules comprenant au moins un polymère et une statine, ladite statine étant isolée du contact avec lesdits esters d'alkyle de PUFA par une membrane polymère qui peut être facilement désintégrée dans le milieu gastro-intestinal.

2. Formulation pharmaceutique selon la revendication 1, **caractérisée en ce que** le PUFA appartient à la série Oméga3.

3. Formulation pharmaceutique selon l'une quelconque des revendications précédentes, **caractérisée en ce que** le PUFA est choisi dans le groupe constitué par l'acide éicosapentaénoïque, l'acide docosahexaénoïque ou des mélanges de ceux-ci.

4. Formulation pharmaceutique selon l'une quelconque des revendications précédentes, **caractérisée en ce que** l'ester d'alkyle de PUFA est choisi dans le groupe constitué par des esters d'éthyle, de méthyle, de propyle, de butyle ou des mélanges de ceux-ci.

5. Formulation pharmaceutique selon l'une quelconque des revendications précédentes, **caractérisée en ce que** les statines sont choisies dans le groupe constitué de simvastatine, lovastatine, fluvastatine, atorvastatine, cérivastatine, pravastatine, rosuvastatine ou des mélanges de celles-ci.

6. Formulation pharmaceutique selon l'une quelconque des revendications précédentes, **caractérisée en ce que** le polymère enrobant les microcapsules de statines est choisi dans le groupe constitué par des polyesters, polyacrylates, polycyanoacrylates, polysaccharides, polyéthylène glycol ou des mélanges de ceux-ci.

7. Formulation pharmaceutique selon l'une quelconque des revendications précédentes, **caractérisée en ce que** le polymère enrobant les microcapsules de statines est choisi dans le groupe constitué de gélatine, carboxyméthylcellulose, alginates, carraghénanes, pectines, éthylcellulose, hydroxypropylméthylcellulose, acétophtalate de cellulose, phtalate d'hydroxypropylméthylcellulose, copolymères d'acide méthacrylique, copolymères de méthacrylate de diméthylaminoéthyle, copolymères de méthacrylate de triméthylammoniuméthyle, copolymères d'acide lactique et glycolique et des copolymères ou des mélanges de ceux-ci.

8. Formulation pharmaceutique selon l'une quelconque des revendications précédentes, **caractérisée en ce qu'**elle comprend en outre un antioxydant.

9. Formulation pharmaceutique selon la revendication 8, **caractérisée en ce que** ledit antioxydant est l'acétate de vitamine E.

10. Formulation pharmaceutique selon l'une quelconque des revendications précédentes, **caractérisée en ce qu'**elle comprend en outre de la carnitine.

11. Formulation pharmaceutique selon l'une quelconque des revendications précédentes, **caractérisée en ce que** les microcapsules représentent entre 1 % et 60 % du poids total de la formulation.

12. Formulation pharmaceutique selon l'une quelconque des revendications précédentes, **caractérisée en ce que** la quantité de statine incorporée dans les microcapsules est comprise entre 1 % et 80 % en poids par rapport au poids total des microcapsules.

13. Formulation pharmaceutique selon la revendication 12, **caractérisée en ce que** ladite quantité de statine incorporée dans les microcapsules est comprise entre 1 et 40 % en poids par rapport au poids total des microcapsules.

14. Formulation pharmaceutique selon l'une quelconque des revendications précédentes, **caractérisée en ce qu'**elle comprend en outre un additif plastifiant du polymère.

15. Formulation pharmaceutique selon la revendication 14, **caractérisée en ce que** ledit additif plastifiant est choisi dans le groupe constitué de citrate de triéthyle, phtalate de butyle et de mélanges de ceux-ci.

16. Formulation pharmaceutique selon l'une quelconque des revendications 3 à 15, **caractérisée en ce que** le rapport entre l'acide éicosapentaénoïque et l'acide docosahexaénoïque est compris entre 0,5 et 2.

17. Formulation selon l'une quelconque des revendications précédentes, **caractérisée en ce que** la suspension des microcapsules est encapsulée par des capsules de gélatine molle pour une administration orale.

18. Formulation selon la revendication 17,
**caractérisée en ce que** les capsules de gélatine molle ont un enrobage gastrorésistant.
